# EUROPEAN PATENT SPECIFICATION

(11) **EP 2 123 168 B1**
(45) Date of publication and mention of the grant of the patent: **21.12.2011**
(21) Application number: 08156353.8
(22) Date of filing: 16.05.2008
(51) Int. Cl.: A23K 1/00, A23L 1/30

(54) **Lactobacillus paracasei and weight control**
Lactobacillus paracasei und Gewichtskontrolle
Lactobacillus paracasei et contrôle de poids

(43) Date of publication of application: 25.11.2009
(73) Proprietor: Nestec S.A., 1800 Vevey (CH)
(72) Inventor: Fukushima, Yoichi, 1815 Clarens (CH)
(74) Representative: Marquardt, Ulf

(56) References cited:
- WO-A-2006/091103
- WO-A-2007/085970
- LEE ET AL: "Human originated bacteria, Lactobacillus rhamnosus PL60, produce conjugated linoleic acid and show anti-obesity effects in diet-induced obese mice" BIOCHIMICA AND BIOPHYSICA ACTA. MOLECULAR AND CELL BIOLOGY OFLIPIDS, ELSEVIER, AMSTERDAM, NL, vol. 1761, no. 7, 1 July 2006 (2006-07-01), pages 736-744, XP005584275 ISSN: 1388-1981
- SENOK ET AL.: "Probiotics: facts and myths", CLIN. MICROBIOL INFECT, vol. 11, 2005, pages 958-966,
- LIU ET AL.: "Antihypertensive effects of Lactobacillus-fermented milk orally administered to spontaneously hypertensive rats.", J. AGRIC. FOOD CHEM., vol. 59, 2011, pages 4537-4543,
- JIA ET AL.: "A 90-day oral toxicity study on a new strain of Lactobacillus paracasei in rats", FOOD AND CHEMICAL TOXICOLOGY, vol. 49, 2011, pages 1148-1151,

## Description

The present invention generally relates to the field of obesity and/or metabolic disorders. In particular the present invention relates to the use of probiotics to treat or prevent overweightness, obesity and/or associated metabolic disorders.

During the past decades, the prevalence of obesity has increased worldwide to epidemic proportion. Approximately 1 billion of people worldwide are overweight or obese, conditions that increase mortality, mobility and economical costs. Obesity develops when energy intake is greater than energy expenditure, the excess energy being stored mainly as fat in adipose tissue. Body weight loss and prevention of weight gain can be achieved by reducing energy intake or bioavailability, increasing energy expenditure and /or reducing storage as fat. Obesity represents a serious threat to health because it is associated with an array of chronic diseases, including diabetes, atherosclerosis, degenerative disorders, airway diseases and some cancers.

Modifications of the intestinal flora were recently associated with obesity. These changes were demonstrated in obese mice to affect the metabolic potential of gut microbiota resulting in an increased capacity to harvest energy from the diet (Tumbaugh PJ, Ley RE, Mahowald MA, Magrini V, Mardis ER, Gordon JI. An obesity-associated gut microbiome with increased capacity for energy harvest. Nature. 2006; Ley RE, Turnbaugh PJ, Klein S, Gordon JI. Microbial ecology: human gut microbes associated with obesity. Nature. 2006). Such modifications of gut microbiota are proposed to contribute to the pathophysiology of obesity. Probiotics, the beneficial bacteria present in food or food supplements, are known to modify the intestinal microbiota (Fuller R & Gibson GR. Modification of the intestinal microflora using probiotics and prebiotics. Scand J. Gastroenterol. 1997).

For example US7001756 and CN1670183 provide an isolated micro-organism strain, Lactobacillus rhamnosus GM-020, which is found to be effective in treating obesity.

However, since some organisms might tolerate the ingestion of some micro-organisms better than the ingestions of others, it would be desirable to have available a variety of micro-organisms that may be chosen based on the needs of the subject to be treated.

WO03055987 discloses a specific Lactobacillus paracasei strain, Lactobacillus paracasei viro-01, isolated and identified from Kimchi fermentation liquid. It may be used as probiotic and decreases diarrhoea incidence.

WO2007015132 discloses a Lactobacillus paracasei strain, I 1688, that can be used for the preparation of immunomodulating compositions, in particular for the treatment and/or prevention of allergies and immunodeficiencies.

WO2006/091103 discloses a composition comprising Bifidobacterium breve, a non-digestible saccharide A and a non-digestible saccharide B, optionally combined with Lactobacillus paracasei. Disclosed is the use of this composition to equalize the bacteria species distribution in the gastrointestinal tract of formula-fed infants.

WO2007/085970 discloses the use of strains of microorganisms and/or metabolites thereof in the manufacture of a support for administration to a subject for modulating satiety signaling. Appropriate strains of "Lactobacillus acidophilus, L. curvatus, L. salivarius and Bifidobacterium lactis were disclosed.

H.Y. Lee et al., Biochimica and Biophysica Acta 1761 (2006) 736-744, disclose a human originated bacterium, Lactobacillus rhamnosus PL60, that produces conjugated linoleic acid and shows anti-obesity effects in diet-induced obese mice.

Based on this prior art it was the object of the present invention to identify alternative probiotic bacteria that can be used to treat or prevent obesity and/or metabolic disorders.

The present inventors were surprised to see that Lactobacillus paracasei can solve the object of the present invention.

One Lactobacillus paracasei strain that is particularly effective for the purpose of the present invention is Lactobacillus paracasei ST11. Lactobacillus paracasei ST11 was deposited under the Budapest treaty with the Institut Pasteur (28 rue du Docteur Roux 75024 Paris Cédex 15) and was designated CNCM I-2116.

The present inventors could show that the probiotic Lactobacillus paracasei affects the autonomic nerve activities and regulates blood glucose and cardiovascular function. The inventors found that an ingestion of Lactobacillus paracasei, for example of Lactobacillus paracasei ST11, reduced body weight and abdominal fat weight. This effect was in particular pronounced after a long term ingestion of Lactobacillus paracasei, in particular Lactobacillus paracasei ST11. The inventors were surprised to see that the ingestion of Lactobacillus paracasei, for example of Lactobacillus paracasei ST11, affects autonomic nerves, enhances lipolysis, and reduces body weight.

Consequently, the object of the present invention is achieved by the use of claim 1.

Hence, one embodiment of the present invention is the use of Lactobacillus paracasei, in particular Lactobacillus paracasei ST11, for the preparation of a composition to overweightness, obesity and/or associated metabolic disorders.

The present invention also relates to a composition comprising Lactobacillus paracasei, in particular Lactobacillus paracasei ST11 for the treatment and/or prevention of overweightness, obesity and/or associated metabolic disorders.

In this specification, the following terms have the following meanings:

The term "Lactobacillus paracasei" is meant to include the bacterium, a cell growth medium with the bacterium or a cell growth medium in which Lactobacillus paracasei was cultivated.

The term "Lactobacillus paracasei ST11" is meant to include the bacterium, a cell growth medium with the bacterium or a cell growth medium in which Lactobacillus paracasei ST11 was cultivated.

"Body mass index" or "BMI" means the ratio of weight in kg divided by the height in metres, squared.

"Overweight" is defined for an adult human as having a BMI between 25 and 30.

"Obesity" is a condition in which the natural energy reserve, stored in the fatty tissue of animals, in particular humans and other mammals, is increased to a point where it is associated with certain health conditions or increased mortality. "Obese" is defined for an adult human as having a BMI greater than 30.

"Probiotic" means microbial cell preparations or components of microbial cells with a beneficial effect on the health or well-being of the host. (Salminen S, Ouwehand A. Benno Y. et al "Probiotics: how should they be defined" Trends Food Sci. Technol. 1999:10 107-10).

"Prebiotic" means food substances that promote the growth of probiotics in the intestines. They are not broken down in the stomach and/or upper intestine or absorbed in the GI tract of the person ingesting them, but they are fermented by the gastrointestinal microflora and/or by probiotics. Prebiotics are for example defined by Glenn R. Gibson and Marcel B. Roberfroid, Dietary Modulation of the Human Colonic Microbiota: Introducing the Concept of Prebiotics, J. Nutr. 1995 125: 1401-1412.

"Food grade micro-organisms" means micro-organisms that are used and generally regarded as safe for use in food.

"Long term administrations" are preferably continuous administrations for more than 6 weeks.

"Short term administrations" are preferably continuous administrations for less than 6 weeks.

The composition prepared by the use of the present invention may be a medicament, a food product, a pet food product, a food additive or a nutraceutical.

The composition of the present invention may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents, gel forming agents, antioxidants and antimicrobials.

The composition may also contain conventional pharmaceutical additives and adjuvants, excipients and diluents, including, but not limited to, water, gelatine of any origin, vegetable gums, ligninsulfonate, talc, sugars, starch, gum arabic, vegetable oils, polyalkylene glycols, flavouring agents, preservatives, stabilizers, emulsifying agents, buffers, lubricants, colorants, wetting agents, fillers, and the like. In all cases, such further components will be selected having regard to their suitability for the intended recipient.

The composition may be a nutritionally complete formula.

The composition according to the invention may comprise a source of protein.

Any suitable dietary protein may be used, for example animal proteins (such as milk proteins, meat proteins and egg proteins); vegetable proteins (such as soy protein, wheat protein, rice protein, and pea protein); mixtures of free amino acids; or combinations thereof. Milk proteins such as casein and whey, and soy proteins are particularly preferred.

The proteins may be intact or hydrolysed or a mixture of intact and hydrolysed proteins. It may be desirable to supply partially hydrolysed proteins (degree of hydrolysis between 2 and 20%), for example for animals believed to be at risk of developing cows' milk allergy. If hydrolysed proteins are required, the hydrolysis process may be carried out as desired and as is known in the art. For example, a whey protein hydrolysate may be prepared by enzymatically hydrolysing the whey fraction in one or more steps. If the whey fraction used as the starting material is substantially lactose free, it is found that the protein suffers much less lysine blockage during the hydrolysis process. This enables the extent of lysine blockage to be reduced from about 15% by weight of total lysine to less than about 10% by weight of lysine; for example about 7% by weight of lysine which greatly improves the nutritional quality of the protein source.

The composition may also contain a source of carbohydrates and/or a source of fat.

If the composition includes a fat source, the fat source preferably provides 5% to 40% of the energy of the composition; for example 20% to 30% of the energy. A suitable fat profile may be obtained using a blend of canola oil, corn oil and high-oleic acid sunflower oil.

A source of carbohydrate may be added to the composition.

The source of carbohydrates preferably provides 40% to 80% of the energy of the composition. Any suitable carbohydrate may be used, for example sucrose, lactose, glucose, fructose, corn syrup solids, maltodextrins, and mixtures thereof. Dietary fibre may also be added if desired. Dietary fibre passes through the small intestine undigested by enzymes and functions as a natural bulking agent and laxative. Dietary fibre may be soluble or insoluble and in general a blend of the two types is preferred. Suitable sources of dietary fibre include soy, pea, oat, pectin, guar gum, gum Arabic, fructooligosaccharides, galacto-oligosaccharides, sialyl-lactose and oligosaccharides derived from animal milks. A preferred fibre blend is a mixture of inulin with shorter chain fructo-oligosaccharides. Preferably, if fibre is present, the fibre content is between 2 and 40 g/l of the composition as consumed, more preferably between 4 and 10 g/l.

The composition may also contain minerals and micronutrients such as trace elements and vitamins in accordance with the recommendations of Government bodies such as the USRDA. For example, the composition may contain per daily dose one or more of the following micronutrients in the ranges given:- 300 to 500 mg calcium, 50 to 100 mg magnesium, 150 to 250 mg phosphorus, 5 to 20 mg iron, 1 to 7 mg zinc, 0.1 to 0.3 mg copper, 50 to 200 µg iodine, 5 to 15 µg selenium, 1000 to 3000 µg beta carotene, 10 to 80 mg Vitamin C, 1 to 2 mg Vitamin B1, 0.5 to 1.5 mg Vitamin B6, 0.5 to 2 mg Vitamin B2, 5 to 18 mg niacin, 0.5 to 2.0 µg Vitamin B12, 100 to 800 µg folic acid, 30 to 70 µg biotin, 1 to 5 µg Vitamin D, 3 to 10 µg Vitamin E.

One or more food grade emulsifiers may be incorporated into the composition if desired; for example diacetyl tartaric acid esters of mono- and di-glycerides, lecithin and mono- and di-glycerides. Similarly suitable salts and stabilisers may be included.

The composition is preferably orally or enterally administrable; for example in the form of a powder for re-constitution with milk or water.

Lactobacillus paracasei, in particular Lactobacillus paracasei ST11, may be cultured according to any suitable method and prepared for addition to the composition by freeze-drying or spray-drying for example. Appropriate culturing methods for Lactobacillus paracasei, in particular Lactobacillus paracasei ST11, are known to those skilled in the art. Alternatively, bacterial preparations can be bought from specialist suppliers.

Generally, the composition of the present invention is intended for infants, children, and/or adults.

In one embodiment of the present invention the subjects to be treated with the composition prepared by the use of the present invention are at least two years old. This age limit applies in particular to humans. If the subject to be treated with the composition prepared by the use of the present invention is a dog or a cat, for example, the dog or cat should be at least 4 months old.

In one embodiment of the present invention the composition is a medicament. As a medicament the dosage of Lactobacillus paracasei, in particular Lactobacillus paracasei ST11, should be carefully adjusted according to a doctor's recommendation.

The composition prepared according to the present invention may also be a food product. As a food product the beneficial effects of Lactobacillus paracasei, in particular Lactobacillus paracasei ST11, would be available to everyone. Treatment of metabolic disorders, overweightness and/or obesity could be initiated at a very early stage and at low costs. Further in a food product Lactobacillus paracasei, in particular Lactobacillus paracasei ST11, would be even more pleasant to consume. Examples of food products that are applicable to the present invention are yoghurts, milk, flavoured milk, ice cream, ready to east desserts, powders for re-constitution with, e.g., milk or water, chocolate milk drinks, malt drinks, ready-to-eat dishes, instant dishes or drinks for humans or food compositions representing a complete or a partial diet intended for pets or livestock.

Consequently, in one embodiment the composition according to the present invention is a food product intended for humans, pets or livestock. In particular the composition is intended for animals selected from the group consisting of dogs, cats, pigs, cattle, horses, goats, sheep, poultry or humans, and in a preferred embodiment is the composition a food product intended for adult species, in particular human adults. Lactobacillus paracasei was found to act also on brown adipose tissue. Brown adipose tissue is a major site of heat production in cold-adapted animals. Consequently, the composition of the present invention is particularly well suited for subjects that have large amounts of brown adipose tissue, such as dogs, for example.

The composition of the present invention may also comprise at least one other kind of other food grade micro-organism, in particular bacteria or yeast. The food grade micro-organism may be a probiotic micro-organism and is preferably selected from the group consisting of lactic acid bacteria, bifidobacteria, propionibacteria or mixtures thereof.

All probiotic micro-organisms may be used in accordance with the present invention. Preferably, they are selected from the group consisting of Bifidobacterium, Lactobacillus, Lactococcus, Enterococcus, Streptococcus and Saccharomyces or mixtures thereof, in particular selected from the group consisting of *Bifidobacterium longum, Bifidobacterium lactis, Lactobacillus acidophilus, Lactobacillus rhamnosus, Lactobacillus paracasei, Lactobacillus johnsonii, Lactobacillus plantarum, Lactobacillus salivarius, Enterococcus faecium, Saccharomyces boulardii* and *Lactobacillus reuteri* or mixtures thereof, preferably selected from the group consisting of *Lactobacillus johnsonii* (NCC533; CNCM I-1225), *Bifidobacterium longum* (NCC490; CNCM I-2170), *Bifidobacterium longum* (NCC2705; CNCM I-2618), *Bifidobacterium lactis* (2818; CNCM I-3446), *Lactobacillus rhamnosus* GG (ATCC53103), *Lactobacillus rhamnosus* (NCC4007; CGMCC 1.3724), *Enterococcus faecium* SF 68 (NCIMB10415), and mixtures thereof

For example probiotics may be also capable of promoting the development of a bifidogenic intestinal microbiota. Suitable probiotic Bifidobacteria strains for this include the Bifidobacteria mentioned above and further *Bifidobacterium lactis* CNCM I-3446 sold *inter alia* by the Christian Hansen company of Denmark under the trade mark Bb12, *Bifidobacterium longum* ATCC BAA-999 sold by Morinaga Milk Industry Co. Ltd. of Japan under the trade mark BB536, the strain of *Bifidobacterium breve* sold by Danisco under the trade mark Bb-03, the strain of *Bifidobacterium breve* sold by Morinaga under the trade mark M-16V and the strain of *Bifidobacterium breve* sold by Institut Rosell (Lallemand) under the trade mark R0070. A mixture of suitable probiotic Lactic acid bacteria and Bifidobacteria may be used.

As food grade yeast Saccharomyces cerevisiae and/or Saccharomyces boulardii can be used for example.

In a preferred embodiment of the present invention the composition further contains at least one prebiotic. Prebiotics can promote the growth of certain food grade bacteria, in particular of probiotic bacteria, in the intestines and can hence enhance the effect of Lactobacillus paracasei, in particular Lactobacillus paracasei ST11. Furthermore, several prebiotics have a positive influence on, e.g., digestion.

The prebiotics that may be used in accordance with the present inventions are not particularly limited and include all food substances that promote the growth of probiotics in the intestines. Preferably, they may be selected from the group consisting of oligosaccharides, optionally containing fructose, galactose, mannose; dietary fibers, in particular soluble fibers, soy fibers; inulin; or mixtures thereof. Preferred prebiotics are fructo-oligosaccharides (FOS), galacto-oligosaccharides (IOS), isomalto-oligosaccharides, xylo-oligosaccharides, oligosaccharides of soy, glycosylsucrose (GS), lactosucrose (LS); lactulose (LA), palatinose-oligosaccharides (PAO), malto-oligosaccharides (MOS), gums and/or hydrolysates thereof, pectins and/or hydrolysates thereof.

One advantage of the present invention is that Lactobacillus paracasei, in particular Lactobacillus paracasei ST11, are effective, both, as living bacterium as well as non replicating bacterial species. Consequently, even conditions that will not allow the presence of living bacteria will not abolish the effectiveness of Lactobacillus paracasei, in particular Lactobacillus paracasei ST11.

It is preferred, however, that at least a part of the Lactobacillus paracasei, in particular Lactobacillus paracasei ST11, are alive in the composition and preferably arrive alive in the intestine. This way they can colonize the intestine and increase their effectiveness by multiplication.

However, for special sterile food products or medicaments it might be preferable that Lactobacillus paracasei, in particular Lactobacillus paracasei ST11, are not alive in the composition. Hence, in one embodiment of the present invention at least a part of the Lactobacillus paracasei, in particular Lactobacillus paracasei ST11 are not alive in the composition.

Lactobacillus paracasei, in particular Lactobacillus paracasei ST11, will be effective in any concentration. If Lactobacillus paracasei, in particular Lactobacillus paracasei ST11, reaches the intestine alive a single bacterium can be sufficient to achieve a powerful effect by colonization and multiplication.

For a composition of the present invention it is generally preferred that a daily dose of the composition comprises between 10² and 10¹² cfu of Lactobacillus paracasei, in particular Lactobacillus paracasei ST11. A particular suitable daily dose of Lactobacillus paracasei, in particular Lactobacillus paracasei ST11, is from 10⁵ to 10¹¹ colony forming units (cfu), more preferably from 10⁷ to 10¹⁰ cfu.

In the case of non replicating Lactobacillus paracasei, in particular Lactobacillus paracasei ST11, it is generally preferred that a daily dose of the composition comprises between 10² and 10¹² cells of Lactobacillus paracasei, in particular Lactobacillus paracasei ST11. A particular suitable daily dose of Lactobacillus paracasei, in particular Lactobacillus paracasei ST11, is from 10⁵ to 10¹¹ cells, more preferably from 10⁷ to 10¹⁰ cells.

For a composition of the present invention it is generally preferred that it comprises between 10³ and 10¹² cfu of Lactobacillus paracasei, in particular Lactobacillus paracasei ST11 per g of the dry weight of the composition. A particular suitable amount of Lactobacillus paracasei, in particular Lactobacillus paracasei ST11, is from 10⁵ to 10¹¹ cfu per g of the dry weight of the composition, more preferably from 10⁷ to 10¹⁰ cfu per g of the dry weight of the composition.

In the case of non replicating Lactobacillus paracasei, in particular Lactobacillus paracasei ST11, it is generally preferred that the composition comprises between 10³ and 10¹² cells of Lactobacillus paracasei, in particular Lactobacillus paracasei ST11, per g of the dry weight of the composition. A particular suitable amount of Lactobacillus paracasei, in particular Lactobacillus paracasei ST11, is from 10⁵ to 10¹¹ cells per g of the dry weight of the composition, more preferably from 10⁷ to 10¹⁰ cells per g of the dry weight of the composition.

A further use of a composition comprising Lactobacillus paracasei, in particular Lactobacillus paracasei ST11, according to the present invention is to support weight loss and/or weight maintenance.

In particular, the composition of the present invention may be used to reduce weight gain, in particular for long term administrations of the composition.

However, in particular for short term administrations the composition of the present invention may be used as a suppressor of food intake.

Consuming the composition of the present invention was found to reduce the total abdominal fat generation, in particular the generation of the mesenteric, the epididymal and/or the perirenal adipose tissues.

The composition of the present invention is in particular well suited to treat or prevent overweightness and/or obesity resulting from a high-fat-diet. A high fat diet is a diet containing at least 110% of the fat contained in a diet as generally recommended.

Since establishing and maintaining a proper body weight and - in particular - an acceptable weight percentage of fat in the body is a key step to treat or prevent metabolic disorders, a further use of a composition comprising Lactobacillus paracasei, in particular Lactobacillus paracasei ST11, according to the present invention is to treat or prevent metabolic disorders, for example such as diabetes, hypertension, liver cirrhosis, metabolic syndrome, and/or cardiovascular diseases. The composition prepared of the present invention can hence make a significant contribution to the well-being of today's population, in particular in well developed countries.

Those skilled in the art will understand that they can freely combine all features of the present invention described herein, without departing from the scope of the invention as disclosed. In particular, features described for the uses of the present invention may be applied to the composition of the present invention and vice versa.

Further advantages and features of the present invention are apparent from the following Examples and Figures.

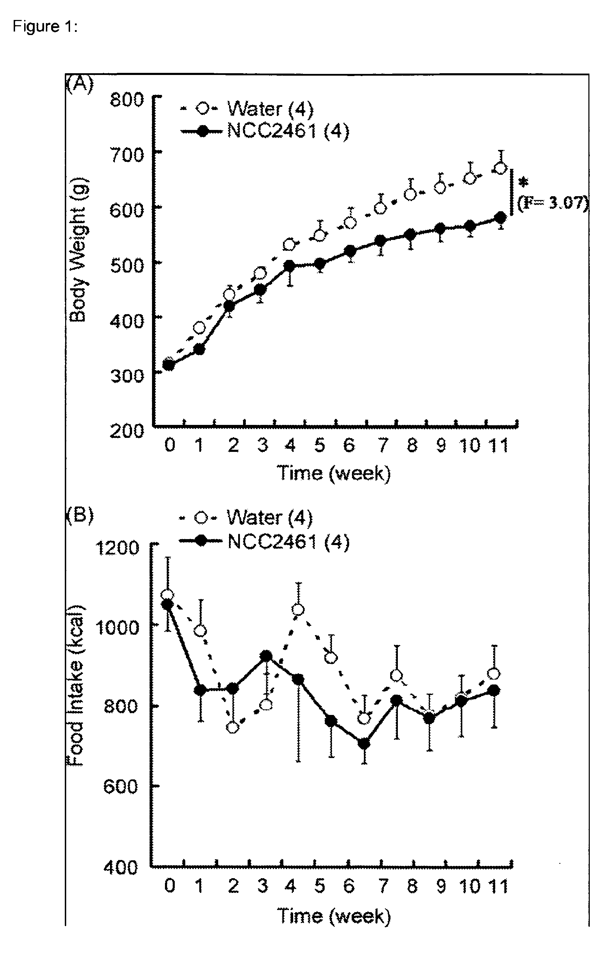

Figure 1 shows the effects of Lactobacillus paracasei ST11 (NCC2461 ) on body weight (A) and food intake (B) fed a high-fat diet for 11 weeks. Values are expressed as mean±S.E.M. in each group. Numbers of animals used are shown in the parentheses. Significant difference between the water and Lactobacillus paracasei ST11 was analyzed by ANOVA. *P < 0.05..

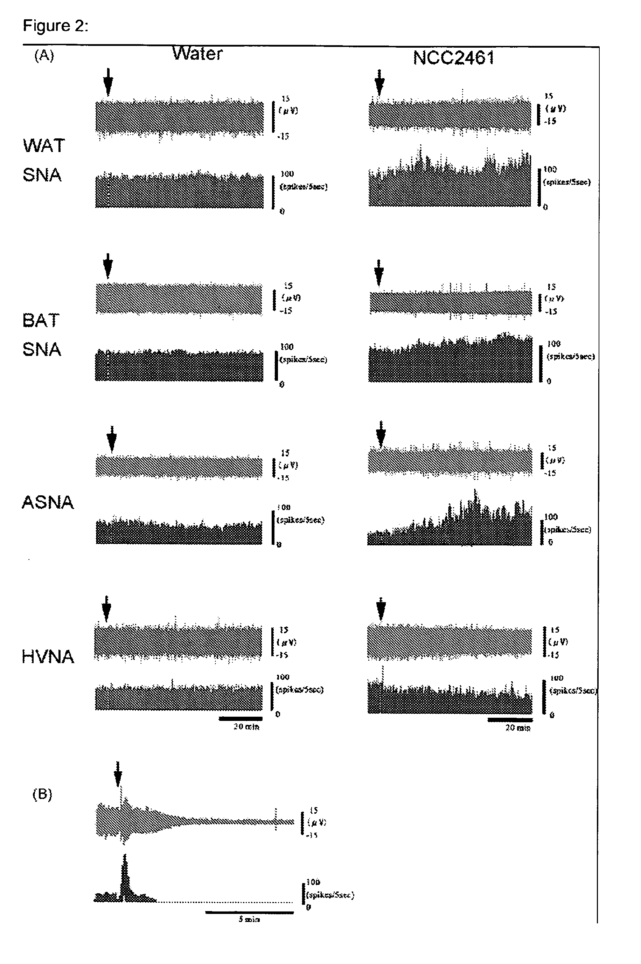

Figure 2 shows data of typical recordings of the WAT-SNA, BAT-SNA, ASNA and HVNA of a rat injected a water or Lactobacillus paracasei ST11 (A). BAT-SNA response to intravenous injection of hexamethonium chloride (B). Injection points are indicated by arrows.

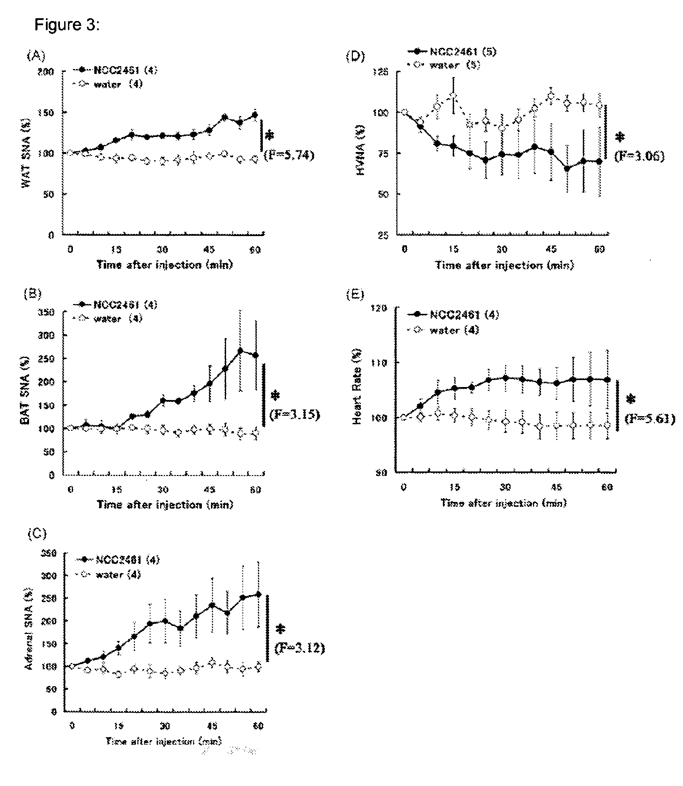

Figure 3 shows the effects of ID injection of Lactobacillus paracasei ST11 (NCC2461) on autonomic nerve activities. WAT-SNA (A),BAT-SNA (B), ASNA (C), HVNA (D) and HR (E) after ID injection of water or Lactobacillus paracasei ST11 are expressed as means±S.E.M. of percentages of values at 0 min. Numbers of animals used are shown in the parentheses. Significant differences (*P < 0.05) between values from 5-60 min after intraduodenal injection of water or Lactobacillus paracasei ST11 are analyzed by ANOVA..

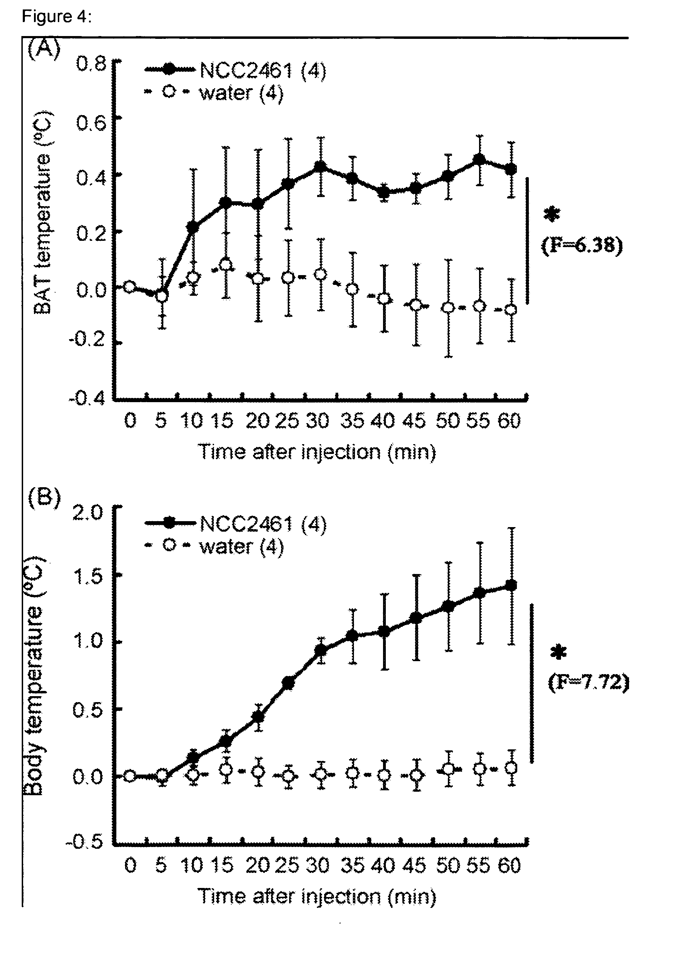

Figure 4 shows the effects of IG injection of Lactobacillus paracasei ST11 (NCC2461) on BAT-T and BT. BAT-T (A) and BT (B) after IG injection of Lactobacillus paracasei ST11 are expressed as means±S.E.M. of percentages of values at 0 min. Numbers of animals used are shown in the parentheses. Significant differences (*P < 0.05) between values from 5 to 60 min after injection in groups are analyzed by ANOVA..

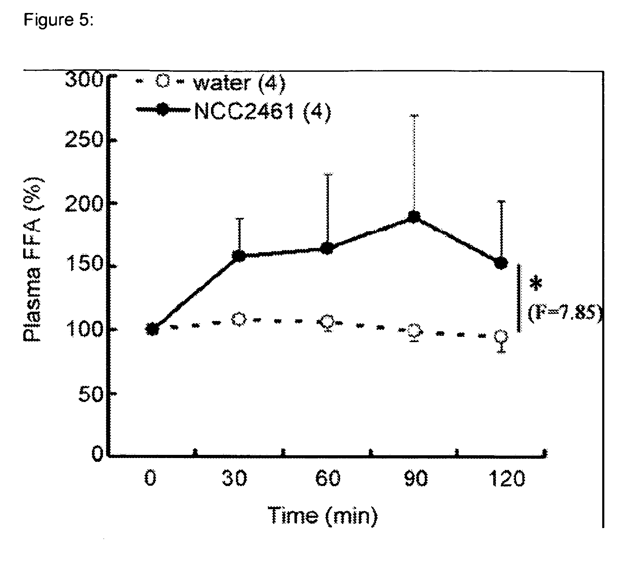

Figure 5 shows changes in plasma FFA after IG injection of Lactobacillus paracasei ST11 (NCC2461). Data (means±S.E.M.) are shown as percentages relative to FFA levels measured at 0 min. Numbers of animals used are shown in the parentheses.
Significant differences in values (*P < 0.05) between the groups from 15 to 60 min are analyzed as groups by ANOVA..

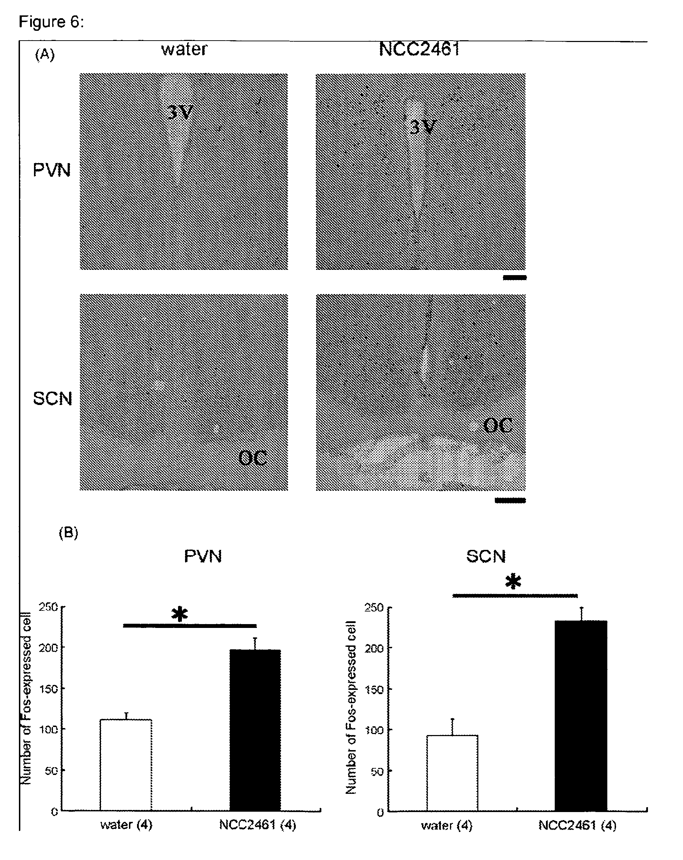

Figure 6 shows the effects of IG injection of Lactobacillus paracasei ST11 (NCC2461) on c-Fos-ir cells in the SCN and the PVN. Representative photomicrographs of coronal sections show the effects of water or Lactobacillus paracasei ST11 on c-Fos-ir cells in the SCN and PVN (A). Abbreviations: 3 V, the third ventricle; OC, the optic chiasm. Scale bars = 200 µm. Number of c-Fos-ir cells in the SCN and PVN after injection of water or Lactobacillus paracasei ST11 was counted (B). Numbers of animals used are shown in the parentheses. The values are expressed as means±S.E.M. *Significant differences are shown as P < 0.05 (Mann-Whitney U-test).

### EXAMPLES:

### Animals

Eight male Wistar rats aged 8 weeks were used and housed individually in a room maintained at 24±1 °C and illuminated for 12 h (07:00-19:00 h) everyday. Rats were adapted to the environment for at least 1 week prior to the experiment and were divided into two drinking groups and give the water or Lactobacillus paracasei ST11 (NCC2461) (1×10⁹ cfu/2 ml water) for 11 weeks. We determined this dose after checking the dose-responses of WAT-SNA in urethane- anesthetized rats (Table 1). The two groups received the high-fat diet [Oriental Yeast Co., Ltd, Tokyo, Japan, dietary composition (% of calorie); 30% fat (beef tallow 14% + lard 14% + soybean oil 2%), casein 25%, corn starch 15%, sucrose 20%, cellulose 5%, mineral mixture (AIN-93G) 4% and vitamin mixture (AIN-93) 1%]. Food and water were freely available. The body weight was monitored in all rats throughout the experimental period, and abdominal fat (mesenteric + epididymal + perirenal adipose tissues) weight was determined after the sacrifice.

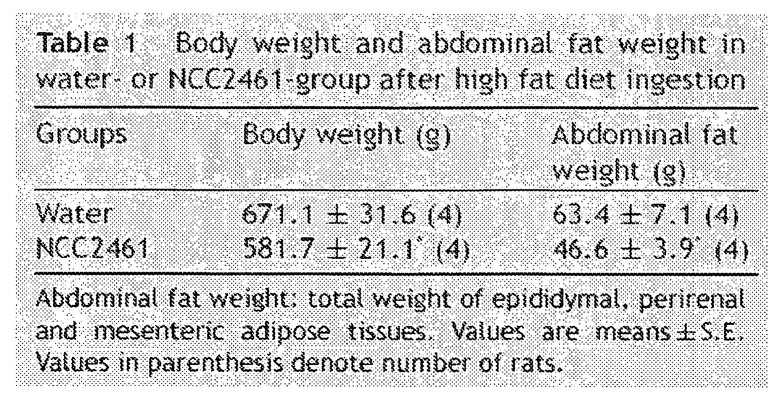

### Electro-physiological study

Male Wistar rats, weighing 300-330 g, were used. Rats were housed in a room maintained at 24±1 °C and illuminated for 12 h (07:00-19:00 h) everyday. Food and water were freely available. Rats were adapted to the environment for at least 1 week prior to the experiment. On the experimental day, food was removed 3-4 h prior to surgery. General preparation was performed as described previously [Tanida M, et al., Brain Res 2005;1058(1-2):44-55], which is herewith incorporated by reference. Briefly, polyethylene catheters were inserted into the left femoral vein and the duodenal cavity for intravenous and intraduodenal injections respectively, under anesthesia induced by intraperitoneal (IP) injection of 1 g/kg urethane. The rats were then cannulated intratracheally, fixed in a stereotaxic apparatus, and maintained at 37.0-37.5 °C. For recording the efferent WAT-SNA (white adipose tissue - synaptic nerve activity), a nerve filament of the sympathetic branch paralleling with the vessel in right WAT of epididymis, was carefully separated under the microscope. For recording the efferent BAT-SNA (brown adipose tissue- synaptic nerve activity), using a dissecting microscope, the left sympathetic nerve innervating interscapular BAT was exposed through a left dorsal incision. For recording adrenal sympathetic nerve activity (ASNA), the left adrenal nerve was exposed retroperitoneally through a left flank incision. For recording hepatic vagal nerve activity, the hepatic branch of the ventral subdiaphragmatic vagal nerve was identified and exposed on the oesphagus after incision of the abdominal midline. The distal end of nerve was ligated, and then hooked to a pair of silver wire electrodes for recording efferent nerve activity. The recording electrodes were immersed in a pool of liquid paraffin oil or a mixture of warm vaseline and liquid paraffin oil for antidehydration of nerves and electrical insulation. The rat was allowed to stabilize for 30-40 min after placement of the recording electrodes. Electrical changes in WAT-SNA, BAT-SNA, ASNA and HVNA (heptic vagal nerve activity) were amplified, filtered and monitored by an oscilloscope. Raw data of the nerve activity was converted to standard pulses by a window discriminator. Two needle electrodes were placed under the skin at the right arm and left leg to record an electrocardiogram and to monitor the heart rate (HR). The signal was amplified with a bioelectric amplifier. These analog signals were converted to digital signals though the A/D converter (Power-Lab model 4sp, AD instruments, USA) sampled and stored on a hard disk for off-line analysis. Baseline measurements of WAT-SNA, BAT-SNA, ASNA, HR and HVNA were made for 5 min before ID injection of Lactobacillus paracasei ST11 (1×10⁹ cfu/2 ml water) or water (2 ml). Lyophilized culture of Lactobacillus paracasei ST11 was used in the experiments after dissolving into water. The indicated parameters were recorded for 60 min after the injection. At the end of the experiment, hexamethonium chloride (10 mg/kg) was intravenously administered to block action potentials of post-ganglionic neural activity in order to determine the noise level of the recording.

### Telemetry recording

To measure the BT (body temperature) and BAT-T (brown adipose tissue - temperature, Telemetry System 181 (Star Medical Co., Japan) was used as described previously [Taniguchi H, et al.,Neurosci Lett 2006;398(1-2):102-6]. In some rats (n = 8), 10-14 days before the intra-gastric (IG) injection of Lactobacillus paracasei ST11, 1 a capsule containing a temperature sensor, battery and transmitter (model 10T-T, Star Medical Co.) was implanted into the abdominal cavity or above the BAT fat pad under pentobarbital anesthesia (35 mg/kg, IP). The output signals mediating a receiver were converted from analog to digital and monitored and stored on a PC. The data obtained by this system were analyzed by 16ch-Eight Star program (Star Medical Corp.). On the experimental day, food was removed 4-6 h before injection. With the animal conscious, baseline measurements of BAT-T or BT were made for 5 min just before IG injection of Lactobacillus paracasei ST11 or water in the light (14:00 h) period. After the injection, these parameters were recorded for 60 min.

### Determination of blood FFA level

Surgical catheterizations were performed under anesthesia with IP pentobarbital 3 days before the experiment. Blood samples were collected from a chronically indwelling silicone catheter implanted in the right external jugular vein, with its end at a point just outside the atrium, and were performed before and after the IG injection of Lactobacillus paracasei ST11 (1×10⁹ cfu/2 ml water) or water (2 ml). The plasma was separated immediately for the assay of FFA (free fatty acids). FFA was assayed using the acyl CoA synthetase-acyl CoA oxidase method with the NEFA-C-test kit (Wako Pure Chemical Industries, Ltd. Cat.# 279-75401).

### Immunohistochemistry

The induction of c-Fos protein in the SCN (suprachiasmatic nucleus) and the PVN was investigated after the IG injections of Lactobacillus paracasei ST11 (1×10⁹ cfu/2 ml water) or water (2 ml). All injections were performed at 14:00 h. 60 min after the injection, rats were anesthetized (pentobarbital 35 mg/kg, IP) and perfused intracardially with ice-cold saline followed by 4% paraformaldehyde (PFA, Sigma, St. Louis, MO) in phosphate-buffered saline (PBS). Brains were removed, postfixed in 4% PFA in PBS at 4 °C overnight, and cryoprotected in 20% sucrose for two nights. The brains were then, sliced using a cryomicrotome (CM1900, Leica, Germany) into 30-µm-thick sections. Immunohistochemical analyses of c-Fos in the SCN and the PVN were conducted using specific polyclonal rabbit antibodies against c-Fos (in 1:1000 dilution; Santa Cruz, South San Francisco, CA) as primary antibodies. Immunoreactivity was visualized with a Vectastain ABC kit (Vector Laboratories, Burlingame, CA) and diaminobenzidine (Sigma, Japan) as the chromogen. To determine the area of the SCN or the PVN, adjacent sections in experimental animals were stained by cresyl violet (Sigma, Japan) and the SCN was delineated. Images of the slices were examined under a microscope (BX51, Olympus, Japan) and the number of c-Fos immunoreactive (c-Fos-ir) nuclei in the SCN or the PVN was counted using Image J program.

### Statistical analysis

WAT-SNA, BAT-SNA, ASNA, HVNA, HR, BAT-T and BT were measured during each 5 min period after Lactobacillus paracasei ST11 injection and analyzed by digital signal processing analyses. All data were expressed as means±S.E.M. a Mann-Whitney U-test was used to compare basal levels in the each groups. Because of the inter-individual variability in the pre-injection state, percent change and temperature change from the baseline were calculated for neural discharge data and temperature data, respectively. Two-way ANOVA was applied to compare group responses of WAT-SNA, BAT-SNA, ASNA, HVNA, HR, BAT-T, BT, body weight and plasma FFA level.

### Results

Fig. 1A shows the changes in body weights of the groups during the experiment. Feeding a HFD (high fat diet) produced progressive increase in body weight in either water or Lactobacillus paracasei ST11 group, but significant reduction of weight gain was confirmed in Lactobacillus paracasei ST11 group. Moreover, drinking a Lactobacillus paracasei ST11 significantly reduced final abdominal fat (mesenteric + epididymal + perirenal adipose tissues) weights as compared to rats that drunk water (Table 2).

**Table 2: Basal levels of autonomic nerve activities, BAT-T and BT in experimental groups**

| Groups | Water | Lactobacillus paracasei ST11 (NCC2461) |
|---|---|---|
| WAT-SNA (spike/5 s) | 198.7 ± 24.2 (4) | 145.2 ± 17.6 (4) |
| BAT-SNA (spike/5 s) | 273.1 ± 72.0 (4) | 155.6 ± 87.7 (4) |
| ASNA (spike/5 s) | 125.6 ± 52.2 (4) | 75.8 ± 25.6 (4) |
| HVNA (spike/5 s)) | 86.5 ± 12.3 (4) | 99.3 ± 20.7 (4) |
| Heart rate (beats/min) | 379.6 ± 24.5 (4) | 360.1 ± 25.5 (4) |
| BAT-T (°C) | 37.7 ± 0.18 (4) | 38.1 ± 0.34 (4) |
| BT (°C) | 36.2 ± 0.28 (4) | 36.2 ± 0.44 (4) |

| | | |
|---|---|---|
| WAT-SNA, white adipose tissue sympathetic nerve activity; BAT-SNA, brown adipose tissue sympathetic nerve activity; ASNA, adrenal sympathetic nerve activity: HVNA, hepatic vagal nerve activity. Data are shown as means±S.E.M. Values in parenthesis denote number of rats. | | |

In addition, significant difference in the long term food consumption during the experimental period between the water group and Lactobacillus paracasei ST11 group was not detected (Fig. 1B). Moreover, significant difference in the total drinking during the whole experimental period between the water group (3248±295 ml) and Lactobacillus paracasei ST11 group 273 (2738±415 ml) was not found. Typical recordings of WAT-SNA, BAT-SNA, ASNA and HVNA in the 60 min following an intestinal injection of Lactobacillus paracasei ST11 are shown in Fig. 2A. Water injection did not affect WAT-SNA, BAT-SNA, ASNA and HVNA, but Lactobacillus paracasei ST11 injection (1×10⁹ cfu) significantly elevated WAT-SNA, BAT-SNA and ASNA, and suppressed HVNA in urethane-anesthetized rats. Moreover, histogram data that intravenous injection of hexamethonium chloride clearly suppressed neural discharge supplying to the BAT are shown in Fig. 2B (pre-injection level; 283.3±90.4 spikes/5 s, level of 10 min after injection; 13.1±5.2 spikes/5 s).

Time-changes in WAT-SNA, BAT-SNA, ASNA, HVNA and HR after injection of Lactobacillus paracasei ST11 or water are summarized in Fig. 3A-E. Lactobacillus paracasei ST11 injection (1×10⁹ cfu) gradually elevated WAT-SNA, which reached a maximum of 147.2±7.3% at 60 min the last time examined (Fig. 3A), BAT-SNA, which ascend to 267.2±88.6% at 55 min (Fig. 3B), ASNA, which ascend to 259.2±72.5% at 60 min the last time examined (Fig. 3C), HR, which ascend to 107.9±4.0% at 50 min the last time examined (Fig. 3E). In addition, Lactobacillus paracasei ST11 injection gradually suppressed HVNA, which reached a minimum of 65.6±14.4% at 50min (Fig. 3D). In contrast, water injection did not significantly affect levels of WAT-SNA, BAT-SNA, ASNA, HR and HVNA, at least up to 60 min after the injection. No significant differences were detected among the 2 groups in the respective basal values at 0 min (Table 3).

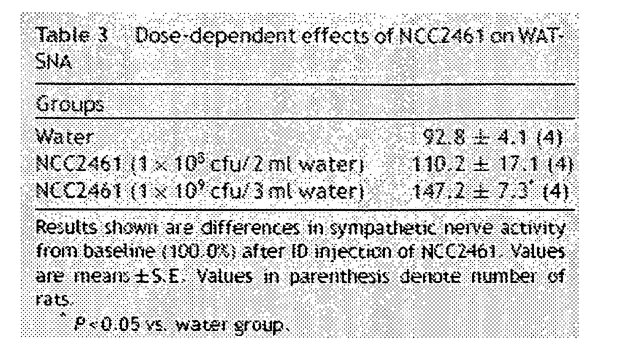

Next, it was examined whether effects of Lactobacillus paracasei ST11 on BAT-T and BT are evoked in conscious rats. As shown in Fig. 4, BAT-T and BT were significantly elevated by IG injection of Lactobacillus paracasei ST11 injection (1×10⁹ cfu) (Fig. 4A and B). Following injection of Lactobacillus paracasei ST11, BAT-T (Fig. 4A) and BT (Fig. 4B) increased gradually, with the greatest levels of elevations occurring at 55 and 60 min, respectively, and these highest levels attained were with 0.45±0.09 and 1.41±0.43 °C, respectively. However, injection of water did not affect BAT-T (Fig. 4A) and BT (Fig. 4B). Absolute basal (0 min) BAT-T and BT values for the experiments shown in Fig. 3 are summarized in Table 3. Differences in respective basal values were not statistically significant (Mann-Whitney U- test).

Finally, the effects of Lactobacillus paracasei ST11 on the c-Fos-ir neurons in hypothalamic nuclei including the SCN and PVN were examined. Fig. 6A depicts representative photomicrographs showing effects of water or Lactobacillus paracasei ST11 on c-Fos-ir cells in the coronally sectioned rat SCN and PVN. Fig. 6B shows the data of c-Fos induction in the SCN and the PVN. The injection of Lactobacillus paracasei ST11 evoked higher expressions of c-Fos-ir cells in both the SCN and the PVN than those by water injection (P < 0.05).

### Results:

In the present study, increases in the body weight and abdominal fat weight of rats fed a HFD were successfully prevented by the ingestion of Lactobacillus paracasei ST11 (NCC2461) as a particular well suited example for Lactobacillus paracasei in general, without changing the food intake.

Moreover, it was found that found that Lactobacillus paracasei, in particular Lactobacillus paracasei ST11, significantly enhances BAT-SNA and WAT-SNA (Fig. 2). In addition, the blood FFA levels, one of lipocatabolic markers, which is stimulated by acceleration of WAT-SNA, were significantly elevated due to the administration of Lactobacillus paracasei ST11 (Fig. 5).

Thus, these data show that Lactobacillus paracasei, in particular Lactobacillus paracasei ST11, suppresses obesity, in particular HFD-induced obesity.

Without wishing to be bound by theory the inventors believe that Lactobacillus paracasei, in particular Lactobacillus paracasei ST11, might affect autonomic nerves and reduces body weight in part or completely via the central histaminergic transduction.

Heat production in the BAT through activation of the sympathetic nerves is mediated uncoupling protein 1, which enhances thermogenesis and energy consumption by uncoupling oxidation from ATP production in mitochondria, and lipolysis in the WAT is induced by triacylglycerol hydrolysis via sympathetic excitation.

The inventors additionally investigated the effects of Lactobacillus paracasei, in particular Lactobacillus paracasei ST11, on BAT-T and plasma FFA level in conscious rats, and obtained data that Lactobacillus paracasei, in particular Lactobacillus paracasei ST11, enhanced BAT-T and plasma FFA level (Figs. 4 and 5). Consequently, Lactobacillus paracasei, in particular Lactobacillus paracasei ST11, might affect autonomic nerves and causes accelerations of thermogenesis and WAT lipolysis.

In present study, the effects of Lactobacillus paracasei, in particular Lactobacillus paracasei ST11, on not only sympathetic, but also parasympathetic nerve activity were analyzed and it was found that HVNA which is implicated in modulations of blood glucose level via glycogenesis in the liver, was suppressed by ingestion of Lactobacillus paracasei, in particular Lactobacillus paracasei ST11, (Fig. 2).

The present study showed that Lactobacillus paracasei, in particular Lactobacillus paracasei ST11, causes sympathetic elevation and parasympathetic suppression. It is known that parasympathetic suppression is linked to food intake inhibition [Shen J,et al., Neurosci Lett 2005;380(3):289-94]. While the present study shows that a long term ingestion of Lactobacillus paracasei, in particular Lactobacillus paracasei ST11, does not affect food intake, possibly due to an adaptation in the gastrointestinal tract, Lactobacillus paracasei, in particular Lactobacillus paracasei ST11, might still be used as a suppressor of food intake, in particular if used for short terms.

## Claims

1. Composition comprising Lactobacillus paracasei ST11 (CNCM I-2116) for use in the treatment or prevention of overweightness, obesity and/or associated metabolic disorders, **characterized in that** the associated metabolic disorder is selected from the group consisting of diabetes, hypertension, liver cirrhosis, metabolic syndrome, cardiovascular diseases, and combinations thereof.

2. Composition in accordance with claim 1, wherein the composition is intended for the treatment of humans or animals, in particular pets, livestock and/or companion animals such as dogs or cats.

3. Composition in accordance with one of claims 1-2, **characterized in that** the composition is a medicament, a food product, a pet food product, a food additive or a nutraceutical.

4. Composition in accordance with one of claims 1-3, **characterized in that** the composition is intended for infants, children, and/or adults.

5. Composition in accordance with claims 1-4, **characterized in that** the composition comprises at least one other kind of other food grade micro-organisms, such as bacteria and/or yeasts.

6. Composition in accordance with claim 5, **characterized in that** the food grade micro-organisms are probiotics.

7. Composition in accordance with claims 1-6, **characterized in that** the composition further contains at least one prebiotic.

8. Composition in accordance with claim 7, **characterized in that** the prebiotic is selected from the group consisting of oligosaccharides and optionally contain fructose, galactose, mannose, soy and/or inulin; dietary fibers; or mixtures thereof.

9. Composition in accordance with one of claims 1-8, **characterized in that** at least a part of the Lactobacillus paracasei are alive in the composition.

10. Composition in accordance with one of claims 1-9, **characterized in that** at least a part of the Lactobacillus paracasei are non replicating in the composition.

11. Composition in accordance with one of claims 1-10, **characterized in that** the composition comprises between 10² and 10¹² cells of Lactobacillus paracasei per daily dose.

12. Composition in accordance with one of claims 1-11 for use in supporting weight loss and/or in supporting weight maintenance, and/or, in particular for short term administrations, in suppressing food intake.

13. Composition in accordance with one of claims 1-12 for use in increasing lipolysis and/or, in particular for long term administrations, in reducing weight gain, in particular while maintaining a constant food intake.

## Patentansprüche

1. Zusammpnsptzung, enthaltend Lactobacillus paracasei ST11 zur Zusammensetzung zur Behandlung oder Vorbeugung von Übergewicht, Adipositas und/oder damit verbundenen metabolischen Störungen, **dadurch gekennzeichnet**, die damit verbundene metabolische Störung ausgewählt ist aus der Gruppe bestehend aus Diabetes, Bluthochdruck, Leberzirrhose, metabolischem Syndrom, Herz-Kreislauf-Erkrankungen, und Kombinationen davon.

2. Zusammensetzung nach Anspruch 1, wobei die Zusammensetzung zur Behandlung von Menschen ober Tieren bestimmt ist, insbesondere von Heimtieren, Nutz- und/oder Haustieren wie zum Beispiel Hunden oder Katzen.

3. Zusammensetzung nach einem der Ansprüche 1-2, **dadurch gekennzeichnet, dass** die Zusammensetzung ein Medikament, ein Nahrungsprodukt, ein Tiernahrungsprodukt, ein Lebensmittelzusatzstoff oder ein Nutraceutical ist.

4. Zusammensetzung nach einem der Ansprüche 1-3, **dadurch gekennzeichnet, dass** die Zusammensptzung für Säuglinge, Kinder und/oder Erwachsene bestimmt sind.

5. Zusammensetzung nach den Ansprüche 1-4, **dadurch gekennzeichnet, dass** die Zusammensetzung mindestens eine weitere Art anderer Mikroorganismen in Lebensmittetquafität, wie zum Beispiel Bakterien und/oder Hefen umfasst.

6. Zusammensetzung nach Anspruch 5, **dadurch gekennzeichnet, dass** die Mikroorganismen in Lebensmittelqualität Probiotika sind.

7. Zusammensetzung nach den Ansprüchen 1-6, **dadurch gekennzeichnet, dass** die Zusammensetzung weiterhin ein Probiotikum enthält.

8. Zusammensetzung nach Anspruch 7, **dadurch gekennzeichnet, dass** das Probiotikum aus der Gruppe bestehend aus Oligosacrhariden ausgewählt ist und gegebenenfalls Fruktose, Galaktose, Mannose, Soja und / ober Inulin, Ballaststoffe, oder Mischungen davon enthält.

9. Zusammensetzung nach einem der Ansprüche 1-8, **dadurch gekennzeichnet, dass** zumindest ein Teil der Lactobacillus paracasei in der Zusammensetzung lebendig sind.

10. Zusammensetzung nach einem der Ansprüche 1-9, **dadurch** gekenntzeichnet, dass zumindest ein Teil der Lactobacillus paracasei in der Zusammensetzung nicht replizierend sind.

11. Zusammensetzung nach einem der Absprüche 1-10, **dadurch gekennzeichnet, dass** die Zusammensetzung zwischen 10² und 10¹² Zellen von Lactobacillus paracasei pro Tagesdosis enthält.

12. Zusammensetzung nach einem der Ansprüche 1-11, zur Unterstützung der Gewichtsabnahme und/oder zur Aufrechterhaltung des Gewichts und/oder, insbesondere für Kunneitanwendungen, zur Unterdrückung der Nahrungsaufnahme.

13. Zusammensetzung nach einem der Anspruche 1-12, zur Steigerung der Lipolyse und/oder, insbesondere für Langzeitanwendungen, zur Reduktion der Gewichtszunahme, insbesondere unter Beibehaltung einer konstanten Nahrungsaufnahme.

## Revendications

1. Composition comprenant du Lactobacillus paracasei ST11 (CNCM I-2116) pour une utilisation dans le traitement ou la prévention du surpoids, de l'obésité et / ou de troubles métaboliques associés, **caractérisée en ce que** le trouble métabolique associé est sélectionné parmi le groupe constitué par le diabète, l'hypertension, la cirrhose du foie, le syndrome métabolique, les maladies cardiovasculaires, et les combinaisons de ceux-ci.

2. Composition conformément à la revendication 1, dans laquelle la composition est destinée au traitement des humains ou des animaux, en particulier des animaux de compagnie, du bétail et / ou des animaux de compagnie comme les chiens ou les chats.

3. Composition conformément à l'une des revendications 1-2, **caractérisée en ce que** la composition est un médicament, un produit alimentaire, un produit alimentaire pour animal de compagnie, un additif alimentaire ou un nutraceutique.

4. Composition conformément à l'une des revendications 1-3, **caractérisée en ce que** la composition est destinée aux nourrissons, aux enfants, et / ou aux adultes.

5. Composition conformément aux revendications 1-4, **caractérisée en ce que** la composition comprend au moins un autre type d'autres micro-organismes de qualité alimentaire, comme des bactéries et / ou des levures.

6. Composition conformément à la revendication 5, **caractérisée en ce que** les micro-organismes de qualité alimentaire sont des probiotiques.

7. Composition conformément aux revendication 1-6, **caractérisée en ce que** la composition contient en outre au moins un prébiotique.

8. Composition conformément à la revendication 7, **caractérisée en ce que** le prébiotique est sélectionné parmi le groupe constitué des oligosaccharides et contient de manière optionnelle du fructose, du galactose, du mannose, du soja et / ou de l'inuline ; des fibres alimentaires ; ou des mélanges de ceux-ci.

9. Composition conformément à l'une des revendications 1-8, **caractérisée en ce qu'**au moins une partie du Lactobacillus paracasei est vivante dans la composition.

10. Composition conformément à l'une des revendications 1-9, **caractérisée en ce qu'**au moins une partie du Lactobacillus paracasei ne se réplique pas dans la composition.

11. Composition conformément à l'une des revendications 1-10, **caractérisée en ce que** la composition comprend entre 10² et 10¹² de cellules de Lactobacillus paracasei par dose quotidienne.

12. Composition conformément à l'une des revendications 1-11 pour une utilisation pour soutenir une perte de poids et / ou pour soutenir un maintien de poids, et / ou, en particulier pour des administrations à court terme, comme suppresseur d'apport alimentaire.

13. composition conformément à l'une des revendications 1-12 pour une utilisation pour augmenter la lipolyse et / ou, en particulier pour des administrations à long terme, pour réduire le gain de poids, en particulier tout en maintenant un apport alimentaire constant.
